# EUROPÄISCHE PATENTSCHRIFT

(11) **EP 3 324 906 B1**
(45) Veröffentlichungstag und Bekanntmachung des Hinweises auf die Patenterteilung: **02.12.2020**
(21) Anmeldenummer: 16736140.1
(22) Anmeldetag: 06.07.2016
(51) Int. Cl.: A61F 9/008, A61B 3/10, A61B 3/14

(54) **POST-OPERATIVE MODIFIKATION EINER INTRAOKULARLINSE**
POSTOPERATIVE MODIFICATION OF AN INTRAOCULAR LENS
MODIFICATION POST-OPÉRATOIRE D'UNE LENTILLE INTRAOCULAIRE

(30) Priorität: 22.07.2015 DE 102015009610
(43) Veröffentlichungstag der Anmeldung: 30.05.2018
(73) Patentinhaber: Carl Zeiss Meditec AG, 07745 Jena (DE)
(72) Erfinder: KRAMPERT, Gerhard, Pleasanton, CA 94566 (US); KINDT, Johannes, 99425 Weimar (DE); BUBLITZ, Daniel, 07646 Rausdorf (DE); POMRAENKE, Robert, 07743 Jena (DE); KÜHNER, Martin, 07639 Bad Klosterlausnitz (DE)
(74) Vertreter: Kintzel, Klaus-Peter
(86) Internationale Anmeldenummer: PCT/EP2016/066030
(87) Internationale Veröffentlichungsnummer: WO 2017/012878

(56) Entgegenhaltungen:
- US-A1- 2003 189 689
- US-A1- 2011 172 649
- US-A1- 2014 257 257
- J. P. Benschop: "Confocal Differential Phase Contrast In Scanning Optical Microscopy", Proceedings of SPIE, vol. 0809, 3 August 1987 (1987-08-03), page 90, XP055444020, US DOI: 10.1117/12.941502 ISBN: 978-1-5106-1533-5

## Beschreibung

Zur Behandlung von Katarakt ist es seit Jahrzehnten üblich die natürliche Linse durch eine künstliche Linse, sogenannte intraokulare Linse, kurz IOL, im Rahmen eines chirurgischen, invasiven Eingriffs zu ersetzen.

Nach dem Einsetzen der intraokularen Linse können aus verschiedensten Gründen jedoch Probleme auftreten, die die Sehfähigkeit des Patienten, bei dem die intraokulare Linse eingesetzt wurde, beeinträchtigen.

So kann, die vor dem Eingriff - durch die gewählte, eingesetzte intraokulare Linse - angestrebte refraktive Korrektur tatsächlich postoperativ nicht erreicht worden sein. Z. B. kann die IOL-Berechnung fehlerhaft erfolgt sein, oder aber die IOL hat sich während des Heilungsprozesses in ihrer Lage und/oder Orientierung im Auge geändert. Dies gilt sowohl für monofokale als auch sogenannte multifokale intraokulare Linsen.

Ferner kann sich nach dem Eingriff herausstellen, dass eine implantierte multifokale Linse vom Patienten z. B. wegen Blendempfindlichkeit nicht vertragen wird.

Oder nach dem Eingriff stellt sich heraus, dass der Patient mit der implantierten monofokalen intraokularen Linse weniger zu Recht kommt, er nun doch sowohl in die Ferne als auch in die Nähe ohne Zuhilfenahme einer Brille sehen möchte, also eine multifokale intraokulare Linse wünscht.

Für alle derartigen Fälle ist eine nachträgliche Änderung der bereits eingesetzten intraokularen Linse ohne weiteren invasiven operativen Eingriff von Vorteil.

Dies kann durch Methoden, Techniken und Aufbauten, welche in den Patentanmeldungen US 2011/172649, US 2009/036880 Kempe, US 2008/001320, US 2009/143858,
US 2009/005764 alle Knox, US 2010/228345, US 2014/135920 beide Bille offenbart sind, realisiert werden. In diesen Anmeldungen wird die Modifikation des Brechungsindex einer bereits implantierten intraokularen Linse durch die Behandlung der Linse mit einem Femtosekunden-Laser beschrieben. Dabei wird auf die genauen Parameter der Laserbehandlung z.B. Pulsenergien, 0,05-1000 nJ, Pulsdauern, 4-100fs, oder auf Parameter des einzuschreibenden Brechungsindex-Muster z. B. typische Änderungen des Brechungsindex der IOL durch den Laser von 0,001 bis 0,03, axiale Ausdehnung des Brechungsindex-Musters von 5-50µm oder die bevorzugte Anwendung eines sogenannten "wrapped lens"-Brechungsindex-Musters eingegangen. Neben dem Problem der Laserparameter und der Art des Index-Musters ist bei der praktischen Anwendung die Lokalisierung der IOL im Auge und/oder auch bereits vorhandener Brechungsindex-Muster in der IOL und Festlegen des Schreib-Musters des Lasers in der IOL, insbesondere bei wiederholter Brechungsindex-Korrektur durch einen Femtosekunden-Laser zu lösen.

Zwar sind Lösungen zum Auffinden der natürlichen Linse schon bekannt z. B. WO04026198 Kempe oder Blumenkranz US2006195076, aber das Auffinden einer IOL mit keiner oder nur geringer Streuung im Volumen der IOL und einem scharfen, lokal begrenzten spekularen Reflex an der IOL-Oberfläche, welcher nicht immer einfach zu erfassen ist, stellt erhöhte Anforderungen.

Zwar erscheint es naheliegend das Schreib-Muster des Lasers, also dass Muster das der Fokuspunkt des Lasers unter Applikation von Laserlicht oder Pulsen abfährt, gleich dem einzuschreibenden Brechungsindex-Muster zu wählen. Dies ist für die erstmalige Korrektur der Fall. Jedoch bei einer Wiederholung einer Brechungsindex-Korrektur kann es sinnvoll sein, wie weiter unten beschrieben, Differenzen zwischen Schreib-Muster und dem noch einzuschreibenden Brechungsindex-Muster zuzulassen.
- Abbildung 1:: System zum Schreiben eines Brechungsindex-Musters in eine implantierte IOL
- Abbildung 2:: Schreib-Muster eines Femtosekunden-Lasers in eine implantierte IOL

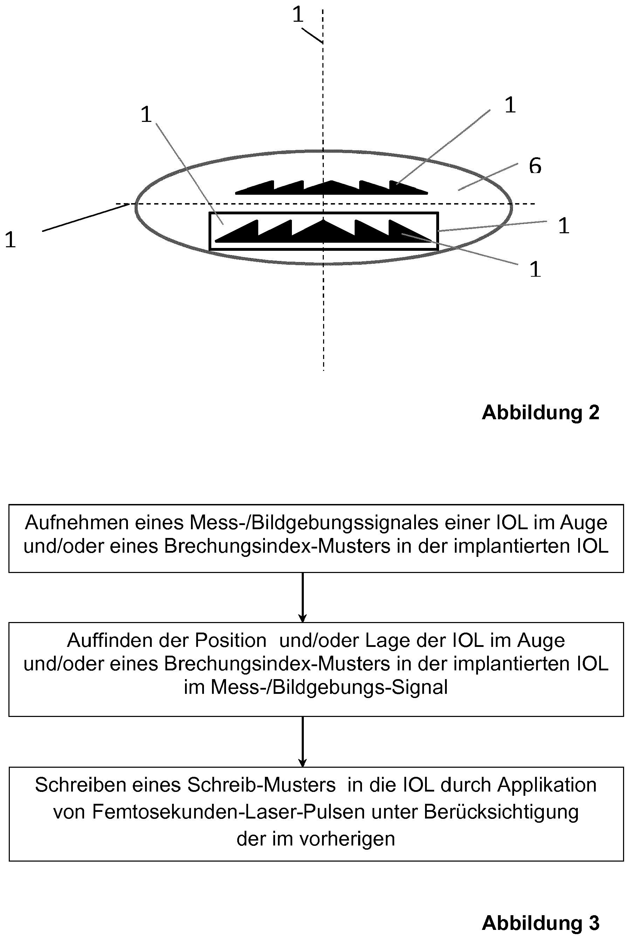

- Abbildung 2:: Schreib-Muster eines Femtosekunden-Lasers in eine implantierte IOL

### Lokalisierung der intraokularen Linse und des Brechungsindex-Musters

Da bei der Berechnung des einzuschreibenden Brechungsindex-Musters von einer nominellen gegebenenfalls vor der Berechnung gemessenen Lage der IOL im Auge ausgegangen wird, muss diese nominelle Lage der IOL auch bei beim Schreiben bekannt sein. Dazu muss vor dem Schreiben des Schreib-Musters in die implementierte IOL zuerst die Lage und/oder Form der intraokularen Linse im Auge bezogen auf das Lasersystem bestimmt werden. Ferner muss geprüft werden, ob für das vorgesehene, nachträglich einzuschreibende Brechungsindex-Muster genügend Raum innerhalb der IOL vorhanden ist. Dies ist insbesondere relevant, wenn der Typ der implementierten IOL nicht bekannt ist, weil der invasive operative Eingriff der Implantation schon zu lange zurück liegt und keine Unterlagen mehr bekannt sind.

Ferner stellt sich für eine bereits schon einmal nichtinvasiv, durch obiges Laserverfahren modifizierte IOL das Problem, dass das vormals eingeschriebene Brechungsindex-Muster in seiner Dimension und/oder Lage in innerhalb der IOL bekannt sein muss. Erst dadurch wird es möglich dieses bereits existierende Brechungsindex-Muster durch ein weiteres Brechungsindex-Muster zu korrigieren. Da der unmodifizierte Brechungsindex der IOL nur wenig von dem Brechungsindex des bereits geschriebenen Brechungsindex-Muster abweicht, ist das Brechungsindex-Muster nur schwer mit klassischen auf Reflektion basierenden Bildgebungsmethoden erkennbar.

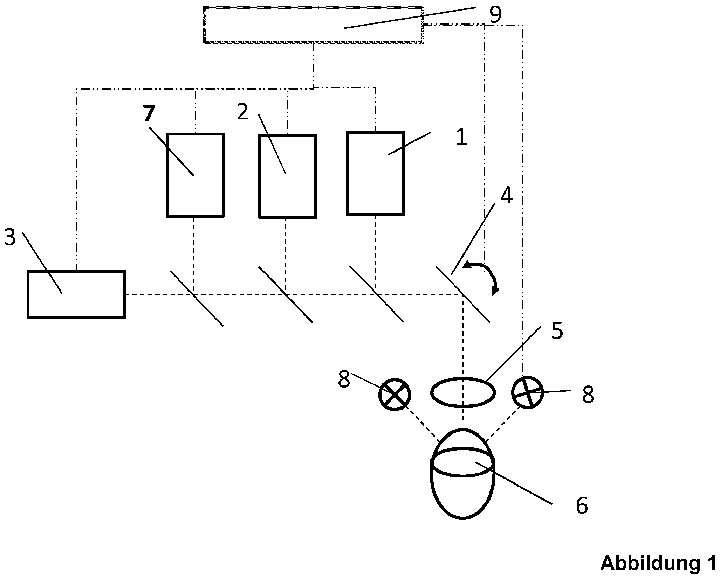

Um für eine Brechungsindex-Modifikation einer IOL durch einen Femtosekunden-Laser die Lage und/oder Form der IOL im Auge und/oder auch die Dimension und/oder Lage eines bereits vorhandenen Brechungsindex-Muster zu identifizieren und die Brechungsindex-Modifikation der IOL durchzuführen wird ein System A aus folgenden Komponenten offenbart - siehe Abbildung 1.
- Femtosekunden-Puls-Lasersystem mit Femtosekunden-Laserquelle 1, einer Optik 5, die den Laserstrahl in das Auge und die IOL 6 fokussiert und einem Scanner 4, welcher den Fokuspunkt des Laserstrahls lateral und axial zur optischen Achse der Optik scannt. In einer Variante ist die Optik selbst ein scannendes Element.
- Optisches-Kohärenz-Tomographie(OCT)-Mess-/Bildgebungs-System mit OCT-Lichtquelle/OCT-Detektor 2 und lateralem Scanner 4, welcher über die gleiche Optik wie der Femtosekunden-Puls-Laser-Strahl das Licht der OCT-Lichtquelle in das Auge leitet und das vom Auge oder seinen inneren Strukturen reflektierte Licht detektiert. In einer Variante ist der Lateral-Scanner mit dem Lateral-Scanner zur Ablenkung des Femtosekunden Lasers identisch. In einer anderen Variante ist das Femtosekunden-Puls-Lasersystem identisch mit der OCT-Lichtquelle.
- Einem Steuergerät 9, welches Signale des OCT-Systems analysiert um die Lage und/oder die Form der IOL im Auge zu erfassen und welches zur Applikation des Schreib-Musters in die IOL das Femtosekunden-Puls-Lasersystem steuert. In einer Ausführungsvariante analysiert das Steuergerät die Signale des OCT-Systems um die Lage und/oder Ausdehnung eines Brechungsindex-Musters innerhalb der IOL in drei Dimensionen zu erfassen.

System A erlaubt eine schnelle Erfassung der IOL und/oder der bereits in der IOL vorhandenen Brechungsindex-Muster, weil der Tiefenscan bei modernen Spektraldomain-OCT-Verfahren wie SweptSource-OCT in kürzerer Zeit als bei den anderen weiter unten vorgestellten Systemen, erfolgen kann. Ferner kann mit dem System A sehr gut ein Brechungsindex-Muster erfasst werden, weil im OCT neben der Änderung des Brechungsindex, welches zu einem Reflexionssignal führt auch noch die Phasenänderung über das Brechungsindex-Muster hinweg, gemessen werden kann. OCT ist für eine solche Phasenänderung sehr empfindlich, während die reine konfokale Detektion gegenüber solchen Phasenänderungen unempfindlich ist.

Alternativ zum obigen System A wird - um für eine Brechungsindex-Modifikation einer IOL durch einen Femtosekunden-Laser die Lage und/oder Form der IOL im Auge und/oder auch die Dimension und/oder Lage eines bereits vorhandenen Brechungsindex-Musters zu identifizieren und die Brechungsindex-Modifikation der IOL durchzuführen - ein System B aus folgenden Komponenten offenbart - siehe Abbildung 1.
- Femtosekunden-Puls-Lasersystem mit Femtosekunden-Laserquelle 1, einer Optik 5 die den Laserstrahl in das Auge und die IOL 6 fokussiert und einem Scanner 4, welcher den Fokuspunkt des Laserstrahls lateral und axial zur optischen Achse der Optik scannt. In einer Variante ist die Optik selbst ein scannendes Element.
- Konfokales-Detektions-Mess-/Bildgebungs-System mit Lichtquelle/Detektor 3 und lateralem Scanner 4, welcher über die gleiche Optik wie der Femtosekunden-Puls-Laser-Strahl das Licht der Lichtquelle in das Auge leitet und das vom Auge oder seinen inneren Strukturen reflektierte Licht detektiert. In einer Variante ist der Lateral-Scanner mit dem Lateral-Scanner zur Ablenkung des Femtosekunden-Lasers identisch.
- Einem Steuergerät 9, welcher Signale des konfokalen-Systems analysiert um die Lage und/oder die Form der IOL im Auge zu erfassen und welcher zur Applikation des Schreib-Musters das Lasersystem steuert. In einer Ausführungsvariante analysiert der Prozessor die Signale des Konfokalen-Systems um die Lage und/oder Ausdehnung eines Brechungsindex-Musters innerhalb der IOL in 3 Dimensionen zu erfassen.

In einer Variante B1 wird der konfokale Detektor des Systems A nach dem Prinzip des Differenzinterferenzkontrastes modifiziert. Dazu wird in den Beleuchtungs-und Detektionsstrahlen ein optisches Element - z. B. ein Wollaston-Prisma eingeschoben, das den Beleuchtungsstrahlengang in zwei nahe beieinanderliegende Strahlen aufteilt und den Detektionsstrahlengang wieder vereint, sodass zwischen beiden Strahlengängen im Detektor eine Interferenz gemessen werden kann. Dadurch ist es möglich Phasenobjekte, wie die des Brechungsindex-Musters lateral zu erkennen, wenn das Objekt, hier die IOL, lateral abgescannt wird. Die Fokuslage entlang der optischen Achse wird durch die Konfokalität in der Detektion erreicht.

In einer Variante B2 wird der konfokale Detektor des Systems A nach dem Prinzip des Phasenkontrastes modifiziert. Dazu werden in den Beleuchtungs- und Detektionsstrahlengang komplementäre Transmissionsfilter und Phasenschieber eingesetzt. Auch dadurch ist es möglich Phasenobjekte, wie die des Brechungsindex-Muster lateral zu erkennen, wenn die IOL lateral abgescannt wird. Die Fokuslage entlang der optischen Achse wird durch die Konfokalität in der Detektion erreicht.

System B hat den Vorteil, dass der Fokus des konfokalen Detektionssystems, weil das Licht des konfokalen Detektionssystems durch die gleiche Optik unter gleichen oder ähnlichen Aperturen wie das Licht des Femtosekunden-Puls-Lasersystems geht, sehr gut mit dem Fokus-Punkt des Femtosekunden-Puls-Lasersystems übereinstimmt. Dies ist insbesondere der Fall wenn als Lichtquelle für das konfokale-Detektionssystem der abgeschwächte und/oder zeitlich gedehnte Femtosekunden-Laserstrahl der Modifikationsstrahlung verwendet wird. Ferner ist die konfokale Detektion aufgrund ihrer großen Detektionsaperturwinkel sehr gut geeignet spekulare Reflexe an gekrümmten, nicht immer senkrecht zur Beleuchtungsachse stehenden Oberflächen, wie die Grenzflächen einer IOL, zu detektieren. Die Varianten System B1 und B2 verbessern das System B dahingehend, dass selbst Phasenobjekte, wie es das Brechungsindex-Muster darstellt gut, detektiert werden können. Allerdings muss zur Lokalisierung des Brechungsindex-Musters innerhalb der IOL ein größerer Bereich konfokal abgescannt werden, was längere Zeit in Anspruch nimmt als die Erfassung durch einen A-Scan im OCT des Systems A.

Alternativ zum obigen System A oder B wird - um für eine Brechungsindex-Modifikation einer IOL durch eine Femtosekunden-Laser die Lage und/oder Form der IOL im Auge und/oder auch die Dimension und/oder Lage des bereits vorhandenen Brechungsindex-Muster zu identifizieren und die Brechungsindex-Modifikation der IOL durchzuführen - ein System C aus folgenden Komponenten offenbart:
- Femtosekunden-Puls-Lasersystem mit Femtosekunden-Laserquelle 1, einer Optik 5, die den Laserstrahl in das Auge und die IOL 6 fokussiert und einem Scanner 4, welcher den Fokuspunkt des Laserstrahls lateral und axial zur optischen Achse der Optik scannt. In einer Variante ist die Optik selbst ein scannendes Element.
- 2D-Mess/Bildgebungs-System bestehend aus einer Kamera 7, die mindestens zwei Aufnahmen unter mindestens zwei unterschiedlichen Beleuchtungswinkeln des Auges und damit der IOL macht und mindestens zwei Lichtquellen 8 zur Erzeugung unterschiedlicher Beleuchtungswinkel. Werden die zwei Aufnahmen gemacht, die das vom Objekt reflektierte Licht detektieren, so kann die Lage und/oder Dimension der IOL im Auge ermittelt werden. Werden zwei Aufnahmen unter Phasenkontrastbildgebung gemacht kann die Lage des Brechungsindex-Muster innerhalb der IOL ermittelt werden. Die laterale Lage und die Lage entlang der optischen Achse kann über Triangulation aus den zwei Aufnahmen bei bekanntem Beleuchtungswinkel in Bezug auf die Kamera ermittelt werden. Besonders einfach wird die Berechnung, wenn sich das Muster zwischen den beiden Aufnahmen nicht verschiebt. Dann liegt das Brechungsindex-Muster im Schnittpunkt der beiden Beleuchtungsachsen- und der Beobachtungsachse sowie im Fokus der Kameradetektion.
- Einem Steuergerät 9, welches Signale des konfokalen Systems analysiert um die Lage und/oder die Form der IOL im Auge zu erfassen und welches zur Applikation des Schreib-Musters das Lasersystem steuert. In einer Ausführungsvariante analysiert das Steuergerät die Signale des OCT-Systems um die Lage und/oder Ausdehnung eines Brechungsindex-Musters innerhalb der IOL in drei Dimensionen zu erfassen.

System C hat den Vorteil gegenüber dem System B und den scannenden Ausführungen des Systems A, dass zur Lokalisierung kein zeitraubendes Abtasten des Objektes notwendig ist. Allerdings ist die laterale und axiale Zuordnung zwischen Bildkoordinaten des Kamerasystems und den Fokuspositionierungslagen des Femtosekunden-Lasersystems komplizierter und muss durch Kalibration an einem Augenphantom gesichert werden.

Um die Nachteile des jeweiligen Systems auszugleichen sind auch Kombinationssysteme denkbar.

Alternativ zum obigen System A, B oder C wird - um für eine Brechungsindex-Modifikation einer IOL durch eine Femtosekunden-Laser die Lage und/oder Form der IOL im Auge und/oder auch die Dimension und/oder Lage des bereits vorhandenen Brechungsindex-Muster zu identifizieren und die Brechungsindex-Modifikation der IOL durchzuführen - ein System D aus folgenden Komponenten offenbart:
- Femtosekunden-Puls-Lasersystem mit Femtosekunden-Laserquelle 1, einer Optik 5, die den Laserstrahl in das Auge und die IOL 6 fokussiert und einem Scanner 4, welcher den Fokuspunkt des Laserstrahls lateral und axial zur optischen Achse der Optik scannt. In einer Variante ist die Optik selbst ein scannendes Element.
- Optisches-Kohärenz-Tomographie(OCT)-Mess-/Bildgebungs-System mit OCT-Lichtquelle/Detektor 2 und lateralem Scanner 4, welcher über die gleiche Optik 5 wie der Femtosekunden-Puls-Laser-Strahl das Licht der OCT-Lichtquelle in das Auge leitet und das vom Auge oder seinen inneren Strukturen reflektierte Licht detektiert. In einer Variante ist der Lateral-Scanner mit dem Lateral-Scanner zur Ablenkung des Femtosekunden Lasers identisch. In einer anderen Variante ist das Femtosekunden-Puls-Lasersystem identisch mit der OCT-Lichtquelle.
- Konfokales-Detektions-Mess-/Bildgebungs-System mit Lichtquelle/Detektor 3 und lateralem Scanner 4, welcher über die gleiche Optik 5 wie der Femtosekunden-Puls-Laser-Strahl das Licht der Lichtquelle in das Auge leitet und das vom Auge oder seinen inneren Strukturen reflektierte Licht detektiert. In einer Variante ist der Lateral-Scanner mit dem Lateral-Scanner zur Ablenkung des Femtosekunden Lasers identisch.
- Einem Steuergerät 9, welches Signale des konfokalen Systems analysiert um die Lage und/oder die Form der IOL im Auge zu erfassen, welches die Signale des OCT-Systems analysiert um die Lage und/oder Ausdehnung eines Brechungsindex-Musters innerhalb der IOL in drei Dimensionen zu erfassen und welcher zur Applikation des Schreib-Musters das Lasersystem steuert.

Durch ein solches System D lässt sich die Schnelligkeit und Robustheit des Auffindens eines Brechungsindex-Musters innerhalb der IOL durch OCT mit der Genauigkeit und Robustheit der Lokalisierung der IOL-Oberflächen durch einen konfokalen Detektor sowie der guten Übereinstimmung zwischen Fokuslagen des konfokalen Detektionssystems und des Femtosekunden-Puls-Lasersystems verbinden.

### Schreib-Muster des Femtosekunden-Puls-Lasers, Brechunqsindex-Muster

Nach der Detektion der intraokularen Linse und/oder eines bereits vorhandenen Brechungsindex-Muster muss festgelegt werden, welches Brechungsindex-Muster nach der Behandlung insgesamt vorliegen soll und wo innerhalb der IOL bzw. wo relativ zu den bereits vorhandenen Brechungsindex-Mustern ein einzuschreibendes Brechungsindex-Muster entstehen soll.

Bei diesem Schreiben eines Brechungsindex-Musters durch einen Femtosekunden-Puls-Laser soll zwischen Schreib-Muster des Laser, dem Muster, das das eingeschaltete Laserlicht bzw. der applizierte Laserpuls beim Scannen in der IOL in einer zusammenhängenden Behandlungsfolge abfährt bzw. austastet und dem dabei erzeugten Brechungsindex-Muster unterschieden werden. Es gibt nämlich IOL-Materialien, die zwar durch eine Laserhandlung lokal ihren Brechungsindex ändern können, indem sich durch Wechselwirkung mit den Laserpulsen die molekulare Struktur lokal ändert und sich z. B. dadurch mehr Wasser als vorher einlagert. Aber bei einer weiteren Laserbehandlung am vormals behandelten Ort ändert sich die molekulare Struktur nur wenig. Es lagert sich auch nicht wesentlich mehr neues Wasser ein und damit ändert sich auch der Brechungsindex nicht weiter. Überstreicht nun ein Schreib-Muster einen vormals bereits erzeugtes Brechungsindex-Muster räumlich exakt, so existiert kein neu eingeschriebenes Brechungsindex-Muster. Überlappen Schreib-Muster und vormals bereits erzeugtes, schon existierendes Brechungsindex-Muster so kommt zum bereits erzeugten Brechungsindex-Muster nur der Teil des Schreib-Musters, welches auf einen vormals nicht beschriebenen IOL-Bereich fällt als neues sogenanntes ergänzendes Brechungsindex-Muster hinzu.

Ferner gibt es Laserparameter/IOL-Material-Kombinationen, die ein Löschen eines vormals geschriebenen, schon existierenden Brechungsindex-Musters bewirken. Dazu ist ein anderes Laserparameterset als beim Schreiben des Brechungsindex-Musters notwendig. Auch beim Löschen kann es zu Sättigungs-Effekten, analog zu den oben beim Schreiben erwähnten kommen.

Für viele Laserparameter/IOL-Material-Kombinationen ist das Schreiben eines Brechungsindex-Musters jedoch irreversibel und nicht ausreichend sättigbar.

Im Folgenden sollen nun Methoden und Methoden-Varianten zur Brechungsindex-Modifikation einer intraokularen Linse durch einen Femtosekunden-Laser-Puls vorgestellt werden. Diese können bevorzugt mit den in vorherigen Abschnitten beschriebenen System A, B, C und D ausgeführt werden.

Es wird offenbart eine Methode zur Brechungsindex-Modifikation einer IOL mittels Femtosekunden-Laser-Pulsen bestehend aus folgenden Schritten - siehe Abbildung 3:
1. Aufnehmen eines Mess-/Bildgebungs-Signales des Auges oder von Strukturen im Auge, insbesondere einer IOL; z. B. in Form eines sogenannten A-Scans oder eines B-Scans, oder auch in 3-Dimensionen durch ein OCT-System oder entsprechende konfokale oder 2D-Bildgebungssignale.
2. Auffinden der Position und/oder Lage der IOL im Auge im Mess-/Bildgebungs-Signal; z.B. durch ein Steuergerät.
3. Schreiben eines Schreib-Musters von Femtosekunden-Laserpulsen in die IOL, - z. B. durch das Femtosekunden-Lasersystem - unter Berücksichtigung der in Schritt 2 gewonnen Daten zur Position und/oder Lage der IOL im Auge, sodass das mit dem Schreib-Muster bewirkte, ggf. ergänzende Brechungsindex-Muster an eine beabsichtigte, nominelle Lage innerhalb der IOL eingeschrieben wird.

Durch das Schreiben eines Schreib-Musters von Femtosekunden-Laserpulsen in die IOL kann - für den Fall, dass die axiale Position der IOL im Auge gegenüber der bei der Implantation geplanten Lage der IOL abweicht - die bereits implantierte IOL in eine IOL mit geänderter Brechkraft umgewandelt werden. Z. B. kann der Fokus einer monofokalen Linse oder aber einer oder mehrere der Foki einer multifokalen Linse axial entlang der optischen Achse der Linse geändert werden.

Durch das Schreiben eines Schreib-Musters von Femtosekunden-Laserpulsen in die IOL kann - für den Fall, dass die Lage der IOL im Auge z. B. die Verkippung der IOL gegenüber einer optischen Achse des Auges oder eine Dezentrierung der IOL gegenüber einer optischen Achse des Auges oder einer Rotation der IOL bezüglich ihrer optischen Achse von der bei der Implantation geplanten Lage abweicht - die bereits implantierte IOL in eine IOL mit zusätzlicher optisch prismatischer Wirkung z. B. durch Schreiben ein keilförmigen oder diffraktiven oder entsprechend wrapped Schreib-Musters ggf. ergänzenden Brechungsindex-Musters umgewandelt werden.

Durch das Schreiben eines Schreib-Musters von Femtosekunden-Laserpulsen in die IOL kann - für den Fall, dass eine bereits implantierte multifokale Linse, nicht vom Patienten vertragen wird - diese in eine monofokale IOL umgewandelt werden.

Durch das Schreiben eines Schreib-Musters von Femtosekunden-Laserpulsen in die IOL kann - für den Fall, dass ein Patient mit bereits implantierter monofokaler IOL nun doch in der Ferne und Nähe sehen will - diese monofokale IOL in eine multifokale IOL umgewandelt werden. Durch das Schreiben eines Schreib-Musters von Femtosekunden-Laserpulsen in die IOL kann - für den Fall, dass ein Patient mit bereits implantierter IOL bei Auftreten oder Fortschreiten einer Retinaerkrankung z. B. einer trockenen Makuladegeneration nach der Implantierung - der Fokus der implantierten Linse in seiner Lage geändert werden. Die Lageänderung des Fokus der IOL kann eine laterale - bzgl. der optischen Achse des Auge - Verschiebung durch Schreiben eines keilförmigen oder diffraktiven oder entsprechend wrapped Schreib-Musters ggf. ergänzenden Brechungsindex-Musters in die IOL sein. Durch die Lageänderung des Fokus der IOL kann der Fokus des Gesamtauges weg von dem durch die Retinaerkrankung betroffenen Areal der Netzhaut in ein noch funktionstüchtiges Areal gelegt werden.

In einer Variante zu Schritt 1 kann das Aufnehmen eines konfokalen Signals und eines OCT-Signales des Auges oder von Strukturen im Auge z. B. durch ein konfokales Detektions-System bzw. ein OCT-System erfolgen. In einer Variante kann zu Schritt 2 ergänzend als Schritt 2.1 das Auffinden der Position und/oder Lage und/oder Dimension eines vorhandenen Brechungsindex-Musters innerhalb der IOL z. B. durch ein Steuergerät hinzukommen. Alternativ oder ergänzend zu Schritt 2.1 kann als Schritt 2.2 das Auffinden von Position und/oder Lage von Markern mit Reflexionskontrast hinzukommen, wobei die Marker in vordefinierter lateraler und axialer Position - z. B. bezogen auf die geometrische Achse der IOL - zu einem vormals eingeschriebenen Brechungsindex-Muster stehen.

In einer Variante können in Schritt 3 folgende Schritte ergänzend hinzukommen:
3.1 Schreiben eines Schreib-Musters von Femtosekunden-Laserpulsen in die IOL, wobei auch die in Schritt 2.1 oder 2.2 gewonnen Daten zur Position und/oder Lage und/oder Dimension eines Brechungsindex-Musters innerhalb der IOL berücksichtigt werden. Dadurch ist es möglich das Schreib-Muster an die beabsichtigte, nominelle Lage relativ zum bereits vorhandenen Brechungsindex-Muster zu setzen.
3.2 Alternativ oder ergänzend zu einem der vorherigen Schritte 3 - siehe auch Abbildung 2: Schreiben eines ersten Schreib-Musters 10 von Femtosekunden-Laserpulsen in eine noch nicht vormals behandelte IOL 9 axial - bzgl. der optischen Achse der IOL - mindestens 200µm posterior zur anterioren IOL-Oberfläche, besonders bevorzugt posterior zur Äquatorachse 11 bzw. posterior zur Mitte der IOL. Dadurch wird erreicht, dass für gegebenenfalls notwendige, weitere Brechungsindex-Modifikationen axial - bzgl. der optischen Achse der IOL - anterior zum ersten eingebrachten Brechungsindex-Muster resultierend aus dem ersten Schreib-Muster genügend Platz für weitere Schreib-Muster oder eine anteriore Ergänzung eines existierenden Brechungsindex-Musters bleibt.
3.3 Alternativ oder ergänzend zu einem der vorherigen Schritte 3: Schreiben eines weiteren Schreib-Musters zur Korrektur eines bisher vorhanden Brechungsindexmusters oder Einschreib-Musters. Dadurch werden Mehrfachkorrekturen z. B. wenn selbst die erste Korrektur aus Patientensicht ungenügend ist möglich. Oder nach einem Schreiben eines ergänzenden Brechungsindex-Musters kann ein diffraktives oder wrapped Schreib-Muster geschrieben werden. Dabei ist besonders bevorzugt das weitere Schreib-Muster axial bzgl. der Augenachse anterior zu den bisher vorhandenen Brechungsindex-Mustern einzuschreiben. Dadurch wird sichergestellt, dass die bisher vorhandenen Brechungsindex-Muster nicht bei der lateralen und axialen Fokussteuerung des Behandlungslasers berücksichtigt werden müssen.
3.4 Alternativ oder ergänzend zu einem der vorherigen Schritte 3: Schreiben eines ersten oder weiteren Schreib-Musters von Femtosekunden-Laserpulsen in die IOL, zur Korrektur einer bisher vorhanden multifokalen Struktur der IOL, höchstens 200µm posterior oder anterior der bisher vorhandenen multifokalen Struktur. Dadurch wird erreicht, dass die bisher vorhandene multifokale Struktur durch das neu eingeschriebene Brechungsindex-Muster in seiner optischen Wirkung korrigiert wird, bevor es zu einer signifikanten und nur schwer zu korrigierenden Überlagerung der Lichtstrahlen verschiedener Ordnungen aus unterschiedlichen Bereichen der multifokalen Struktur kommt.
3.5 Alternativ oder ergänzend zu einem der vorherigen Schritte 3 -siehe Abbildung 2: Schreiben eines Schreib-Musters von Femtosekunden-Laserpulsen - umfasst durch Rand 11 - um schon vorab existierende Brechungsindex-Muster 10 herum oder einbettend. Dadurch entsteht um den vorab existierendes Brechungsindex-Muster ein ergänzendes Brechungsindex-Muster 12. Vorteilhaft ist dabei, wenn durch das ergänzende Brechungsindex-Muster 12 ein vorab vorhandenes diffraktives oder wrapped Brechungsindex-Muster 10 auf ein einfach geometrisches Brechungsindex-Muster - mit Rand 11 -, z. B. Quader oder Linsen ergänzt oder aufgefüllt wird. Solch einfache geometrischen Muster, deren optische Wirkung aus vorhandenem und ergänztem Brechungsindex-Muster nicht diffraktiv und nicht einer "wrapped lens" entspricht, ist einfacher beim Schreiben eines weiteren Schreib-Musters zu berücksichtigen; sowohl bei der Berechnung der Laserfokus-Lagen - welche durch diffraktive oder wrapped Strukturen gestört werden können -, als auch bei der Auswahl eines weiteren Brechungsindex-Musters um die gewünschte Gesamtrefraktion der IOL zu erreichen. Solches Auffüllen auf einfache geometrische Muster ist besonders vorteilhaft für das Schreiben eines weiteren, diffraktiven oder wrapped Brechungsindex-Musters 13, wegen der höheren Toleranz bzgl. axialen und lateralen Lage - bzgl. der optischen Achse des Auges - des diffraktiven oder wrapped Musters zum einfachen geometrischen Muster gegenüber der engeren Toleranz der Lagen zwischen zwei diffraktiven Mustern, wenn kein Auffüllen erfolgt. Für sättigende Laserparameter/Material-Kombinationen ist es besonders vorteilhaft, wenn sich Schreib-Muster und schon vorhandenes Brechungsindex-Muster überlappen. In diesem Fall kann das Schreibmuster, welches durch den Rand 11 gekennzeichnet ist auch den Bereich des schon vorher existierenden Brechungsindex-Musters 10 ganz oder teilweise abdecken. Für diese Kombinationen ändert sich durch die zweite Behandlung im überlappenden Bereich der Brechungsindex nicht weiter. Dies ist vorteilhaft, weil in robuster Art und Weise eine zusammenhängendes und lückenloses Brechungsindex-Muster mit Rand 11 entsteht und weil in diesen Fall auf eine lateral und axial exakte und daher zeitaufwendige Positionierung eines weiteren Schreibmusters 13 in Bezug auf das vor dem weiteren Schreibmuster vorhandene Brechungsindex-Musters 11 verzichtet werden kann. Dabei kann das weitere Schreibmuster 13 mit oder ohne Abstand zum Brechungsindex-Muster 11 geschrieben werden.
3.6 Alternativ oder ergänzend zu einem der vorherigen Schritte 3: Schreiben eines Schreib-Musters von Femtosekunden-Laserpulsen auf ein vorab existierende Brechungsindex-Muster, wobei die Laserparameter so gewählt sind, dass der existierende Brechungsindex auf den ursprünglichen Brechungsindex zurückgesetzt wird - sogenanntes löschendes Schreib-Muster. Auch hierdurch wird erreicht, dass schon z.B. vorab vorhandene diffraktive oder wrapped Brechungsindex-Muster zurückgesetzt werden, sodass diese beim Schreiben eines weiteren Brechungsindex-Musters nicht mehr zu berücksichtigen sind. Für sättigende Laserparameter/Material-Kombinationen ist es besonders vorteilhaft, wenn das Schreib-Muster das schon vorhandene Brechungsindex-Muster komplett überdeckt und zusätzlich auch Teile des vormals unbeschriebenen IOL-Bereichs, quasi über den Rand des schon vorhandenen Brechungsindexmusters hinaus, abdeckt. Dies ist vorteilhaft, weil in diesen Fall in robuster Art und Weise eine IOL mit ursprünglichen, homogenen Brechungsindex entsteht und auf eine lateral und axial exakte und daher zeitaufwendige Positionierung eines weiteren Schreibmusters 13 in Bezug auf das vor dem weiteren Schreibmuster vorhandene Brechungsindex-Musters 11 verzichtet werden kann.
3.7 Alternativ oder ergänzend zu einem der vorherigen Schritte 3: Schreiben eines Reflexionsmarkers in die IOL z. B. unter Verwendung von Femtosekunden- Laser-Parameter, die zu irreversiblen Schnitten in der IOL führen und die einen festen lateralen und axialen - bzgl. der geometrischen Achse der IOL - Bezug zu den in Schritt 3 geschriebenen Schreib-Mustern haben.

Mit dem besonders bevorzugten System D lässt sich darüber hinaus folgende besonders bevorzugte Methode zur Brechungsindex-Modifikation einer intraokularen Linse durch einen Femtosekunden-Laser-Puls durchführen.

Es wird offenbart eine Methode B zur Brechungsindex-Modifikation einer IOL mittels Femtosekunden-Laser-Pulsen bestehend aus folgenden Schritten:
1. Aufnehmen eines konfokalen Signals und eine OCT-Signales des Auges oder von Strukturen im Auge z. B. durch das konfokale Detektions-System bzw. das OCT-System.
2. Auffinden der Position und/oder Lager der IOL im Auge bzgl. des Koordinatensystems des konfokalen Detektions-Systems im konfokalen Signal. Auffinden der Position und/oder Lage der IOL im Auge bzgl. des Koordinatensystems des OCT-Systems im OCT-Signal. Berechnung einer Koordinatentransformation der IOL-Position/Lagedaten gewonnen für das Koordinatensystem des OCT-Systems auf die IOL-Position/Lagedaten gewonnen für das Koordinatensystem des konfokalen Systems. Auffinden der Position und/oder Lage und/oder Dimension eines Brechungsindex-Musters innerhalb der IOL bezogen auf das Koordinatensystem des OCT-Systems im OCT-Signal. Umrechnung der Lage des Brechungsindex-Musters im Koordinatensystem des OCT-Systems anhand der Koordinatentransformation auf eine Lage bzgl. des Koordinatensystems des konfokalen Detektionssystems. Diese Berechnungen könne z. B. durch eine Steuergerät erfolgen.
3. Schreiben eines Schreib-Musters von Femtosekunden-Laserpulsen in die IOL, z. B. durch das Femtosekunden-Lasersystem, unter Berücksichtigung der in Schritt 2 gewonnen Daten zur Position und/oder Lager der IOL im Auge aus dem konfokalen Signal und unter Berücksichtigung der in Schritt 3 gewonnenen Daten zur Position/Lage/Dimension des Brechungsindex-Musters bzgl. des Koordinatensystems des konfokalen Detektions-Systems. Durch die Transformation auf Koordinaten bzgl. des konfokalen Detektions-System anhand des konkreten Auges wird besser als bei einer Kalibrierung anhand eines Augenphantoms sichergestellt, dass das Schreib-Muster an die beabsichtigte, nominelle Lage innerhalb der IOL geschrieben wird.

Auch für dieses Methode B sind als Varianten, die Varianten möglich die bereits als Varianten für Methode A aufgeführt sind. Insbesondere sind für Methode B als Variante die Varianten 3.2-3.7 möglich.

Die Erfindung wird in den Ansprüchen definiert. Weitere Ausführungsbeispiele, Methoden usw. sind keine Teile der Erfindung.

### Bezugszeichenliste

- 1: Femtosekunden-Lichtquelle
- 2: OCT-Lichtquelle und OCT-Detektor
- 3: Lichtquelle und Detektor für konfokales System
- 4: Scan-Spiegel
- 5: Fokussieroptik
- 6: IOL im Auge
- 7: 2D-Kamera
- 8: Spaltprojektor
- 9: Steuergerät
- 10: vormals geschriebenes Brechungsindex-Muster
- 11: äußerer Rand des Schreib-Musters
- 12: ergänztes Brechungsindex-Muster
- 13: neues Schreib-Muster
- 14: Symmetrieachse der IOL entlang der optischen Achse des Auges
- 15: Äquatorialachse der IOL

## Patentansprüche

1. System zur Brechungsindex-Modifikation einer bereits mittels Laserverfahren modifizierten IOL durch einen Femtosekunden-Laser bestehend aus:
• einem Femtosekunden-Puls-Lasersystem mit Femtosekunden-Laserquelle, einer Optik, die geeignet ist, den Laserstrahl in das Auge und die IOL zu fokussieren, und einem Scanner, welcher geeignet ist, den Fokuspunkt des Laserstrahls lateral und axial zur optischen Achse der Optik zu scannen;
• einem Optischen-Kohärenz-Tomographie(OCT)-Mess-/Bildgebungs-System mit OCT-Lichtquelle/Detektor und lateralem Scanner, welcher geeignet ist, das Licht der OCT-Lichtquelle über die gleiche Optik wie den Femtosekunden-Puls-Laser-Strahl in das Auge zu leiten, wobei der OCT-Detektor geeignet ist, aus dem vom Auge oder seinen inneren Strukturen reflektierten Licht ein Brechungsindex-Muster und die Phasenänderung über dieses Brechungsindex-Muster zu detektieren;
**gekennzeichnet durch** ein
Steuergerät, welches geeignet ist, Signale des OCT-Systems zu analysieren, um sowohl die Lage und/oder die Form der IOL im Auge, als auch die Dimension und/oder Lage eines bereits vorhandenen Brechungsindex-Musters zu erfassen, ein
Schreib-Muster zu generieren, wobei sowohl die Lage und/ oder die Form der IOL im Auge, als auch die Dimension und/oder Lage eines bereits vorhandenen Brechungsindex-Musters berücksichtigt werden; und das Femtosekunden-Puls-Lasersystem zur Applikation des Schreib-Musters in die IOL zu steuern.

2. System zur Brechungsindex-Modifikation einer bereits mittels Laserverfahren modifizierten IOL durch einen Femtosekunden-Laser bestehend aus:
• einem Femtosekunden-Puls-Lasersystem mit Femtosekunden-Laserquelle, einer Optik, die geeignet ist, den Laserstrahl in das Auge und die IOL zu fokussieren, und einem Scanner, welcher geeignet ist, den Fokuspunkt des Laserstrahls lateral und axial zur optischen Achse der Optik zu scannen;
• einem Konfokalen-Detektions-Mess-/Bildgebungs-System mit Lichtquelle/Detektor und lateralem Scanner, welcher geeignet ist, das Licht der Lichtquelle über die gleiche Optik wie den Femtosekunden-Puls-Laser-Strahl in das Auge zu leiten, das vom Auge oder seinen inneren Strukturen reflektierte Licht vom Detektor detektiert wird, wobei im konfokalen Detektions-System zusätzliche optische Elemente zur Realisierung eines differenziellen Interferenz-Kontrastes oder eines Phasenkontrastes vorhanden sind
**gekennzeichnet durch** ein
Steuergerät, welches geeignet ist, Signale des konfokalen Systems zu analysieren, um sowohl die Lage und/oder die Form der IOL im Auge, als auch die Dimension und/oder Lage eines bereits vorhandenen Brechungsindex-Musters zu erfassen, ein Schreib-Muster für die IOL zu generieren, wobei sowohl die Lage und/oder die Form der IOL im Auge, als auch die Dimension und/oder Lage eines bereits vorhandenen Brechungsindex-Musters berücksichtigt werden; und das Lasersystem zur Applikation des Schreib-Musters in die IOL zu steuern.

3. System zur Brechungsindex-Modifikation einer bereits mittels Laserverfahren modifizierten IOL durch einen Femtosekunden-Laser bestehend aus:
• einem Femtosekunden-Puls-Lasersystem mit Femtosekunden-Laserquelle, einer Optik, die geeignet ist, den Laserstrahl in das Auge und der IOL zu fokussieren, und einem Scanner, welcher geeignet ist, den Fokuspunkt des Laserstrahls lateral und axial zur optischen Achse der Optik zu scannen,
**gekennzeichnet durch** ein 2D-Mess/Bildgebungs-System bestehend aus einer Kamera, die geeignet ist, mindestens zwei Aufnahmen unter mindestens zwei unterschiedlichen Beleuchtungswinkel des Auges und damit der IOL zu machen und 2 Beleuchtungsquellen zur deren Beleuchtung, wobei das 2D-Mess/Bildgebungssystem zusätzlich optische Elemente zur Realisierung eines Phasenkontrastbildes enthält und
• ein Steuergerät, welches geeignet ist, Signale der Kamera zu analysieren, um sowohl die Lage und/oder die Form der IOL im Auge, als auch die Dimension und/oder Lage eines bereits vorhandenen Brechungsindex-Musters zu erfassen, ein Schreib-Muster für die IOL zu generieren, wobei sowohl die Lage und/oder die Form der IOL im Auge, als auch die Dimension und/oder Lage eines bereits vorhandenen Brechungsindex-Musters berücksichtigt werden; und
das Lasersystem zur Applikation des Schreib-Musters in die IOL zu steuern.

4. System zur Brechungsindex-Modifikation einer bereits mittels Laserverfahren modifizierten IOL durch einen Femtosekunden-Laser bestehend aus:
• einem Femtosekunden-Puls-Lasersystem mit Femtosekunden-Laserquelle, einer Optik, die geeignet ist, den Laserstrahl in das Auge fokussieren, und einem Scanner, welcher geeignet ist, den Fokuspunkt des Laserstrahls lateral und axial zur optischen Achse der Optik zu scannen;
• einem Optischen-Kohärenz-Tomographie(OCT)-Mess-/Bildgebungs-System mit OCT-Lichtquelle/Detektor und lateralem Scanner, welcher geeignet ist,
das Licht der OCT-Lichtquelle über die gleiche Optik wie den Femtosekunden-Puls-Laser-Strahl in das Auge zu leiten, und der OCT-Detektor geeignet ist, das vom Auge oder seinen inneren Strukturen reflektierte Licht zu detektieren;
• einem Konfokalen-Detektions-Mess-/Bildgebungs-System mit Lichtquelle/Detektor und lateralem Scanner, welcher geeignet ist, das Licht der Lichtquelle über die gleiche Optik wie den Femtosekunden-Puls-Laser-Strahl in das Auge leiten; und der Detektor geeignet ist, das vom Auge oder seinen inneren Strukturen reflektierte Licht zu detektieren;
**gekennzeichnet durch** ein
Steuergerät, welches geeignet ist, Signale des konfokalen System zu analysieren, um die Lage und/oder die Form der IOL im Auge zu erfassen, und die Signale des OCT-Systems zu analysieren um die Lage, Ausdehnung eines Brechungsindex-Musters innerhalb der IOL in 3 Dimensionen zu erfassen,
wobei des Steuergerät ferner geeignet ist, ein Schreib-Muster
zu generieren, wobei sowohl die Lage und/oder die Form der IOL im Auge, als auch die Ausdehnung und Lage eines bereits vorhandenen Brechungsindex-Musters berücksichtigt werden; und das Lasersystem zur Applikation des Schreib-Musters in die IOL zu steuern.

5. System nach Anspruch 4, wobei die Optik des Femtosekunden-Puls-Lasersystems mit Femtosekunden-Laserquelle selbst ein scannendes Element ist.

6. System nach Anspruch 4, wobei der laterale Scanner des optischen-Kohärenz-Tomographie(OCT)-Mess-/Bildgebungs-Systems mit dem lateralen Scanner zur Ablenkung des Femtosekunden Lasers identisch ist.

7. System nach einem der Ansprüche 1 bis 4, wobei die Steuereinheit ausgebildet ist, das Lasersystem so zu steuern, dass ein erstes oder weiteres Schreib-Muster von Femtosekunden-Laserpulsen in die IOL, zur Korrektur einer bisher vorhanden multifokalen Struktur der IOL, höchstens 200µm posterior oder anterior der bisher vorhandenen multifokalen Struktur appliziert wird.

8. System nach einem der Ansprüche 1 bis 4, wobei die Steuereinheit ausgebildet ist, das Lasersystem in Abhängigkeit des IOL-Materials so zu steuern, dass ein weiteres Schreib-Muster zur Korrektur eines bisher vorhanden Brechungsindex-Musters appliziert wird.

9. System nach Anspruch 8, wobei die Steuereinheit ausgebildet ist, das Lasersystem so zu steuern, dass weitere Schreib-Muster axial bzgl. der Augenachse anterior zu den bisher vorhandenen Brechungsindex-Mustern appliziert werden.

10. System nach Anspruch 8, wobei die Steuereinheit ausgebildet ist, das Lasersystem so zu steuern, dass weitere Schreib-Muster um bereits existierende Brechungsindex-Muster herum oder in diese eingebettet appliziert werden.

11. System nach Anspruch 10, wobei die Steuereinheit ausgebildet ist, das Lasersystem so zu steuern, dass das vorhandene Brechungsindex-Muster und das ergänzte Schreib-Muster zusammen ein geometrisch einfaches, zusammenhängendes Brechungsindex-Muster mit nicht diffraktiver und nicht einer wrapped lens entsprechender optischen Wirkung ergeben.

12. System nach Anspruch 10, wobei die Steuereinheit ausgebildet ist, das Lasersystem so zu steuern, dass sich das Schreib-Muster und das vorhanden Brechungsindex-Muster überlappen.

13. System nach Anspruch 8, wobei die Steuereinheit ausgebildet ist, das Lasersystem so zu steuern, dass das vorhandene Brechungsindex-Muster durch das Schreib-Muster gelöscht wird.

14. System nach Anspruch 8, wobei die Steuereinheit ausgebildet ist, das Lasersystem so zu steuern, dass das Schreib-Muster das vorhandene Brechungsindex-Muster komplett überdeckt und zusätzlich auch Teile des vormals unbeschriebenen IOL-Bereichs, abdeckt.

15. System nach Anspruch 4, wobei die Steuereinheit ausgebildet ist,
• OCT-Signale zur Berechnung der Lage des vorhandenen Brechungsindex-Musters und der Lage der IOL bzgl. des Koordinatensystem eines OCT-Systems zu verwenden,
• konfokale Detektions-Signale zur Berechnung der Lage der IOL bzgl. des Koordinatensystems eines konfokalen Detektions-Systems zu verwenden,
• eine Koordinatentransformation aus der IOL-Lage bzgl. des Koordinatensystems des OCT-Systems und der IOL-Lage bzgl. des Koordinatensystem des konfokalen Detektions-Systems zu berechnen,
• die Lage des vorhandenen Brechungsindex-Musters bzgl. des Koordinatensystems des OCT-Systems anhand der Koordinatentransformation in eine Lage des vorhandenen Brechungsindex-Musters bzgl. Koordinatensystems des konfokalen Detektions-Systems umzurechnen und
• zur Berechnung des Schreib-Musters Lagedaten der IOL und des vorhandenen Brechungsindex-Musters bzgl. Koordinatensystems des konfokalen Detektionssystems zu berücksichtigen.

16. System nach einem der Ansprüche 1 bis 4, wobei die Steuereinheit ausgebildet ist, ein oder mehrere Schreib-Muster zu erzeugen und zu applizieren, um
• eine monofokale IOL in eine multifokale IOL oder
• eine multifokale IOL in eine monofokale IOL oder
• eine nicht-prismatische IOL in eine IOL mit zusätzlichem prismatischem Effekt umzuwandeln.

## Claims

1. System for modifying the refractive index of an IOL, which has already been modified by means of a laser method, by a femtosecond laser, consisting of:
• a femtosecond pulse laser system comprising a femtosecond laser source, an optical unit suitable for focusing the laser beam into the eye and the IOL, and a scanner suitable for scanning the focal point of the laser beam laterally and axially with respect to the optical axis of the optical unit;
• an optical coherence tomography (OCT) measurement/imaging system comprising an OCT light source/detector and a lateral scanner suitable for guiding the light of the OCT light source into the eye via the same optical unit as the femtosecond pulse laser beam, wherein the OCT detector is suitable for detecting a refractive index pattern and the phase change over this refractive index pattern from the light reflected by the eye or the internal structures thereof,
**characterized by**
a controller suitable for analysing the signals of the OCT system in order to capture both the relative position and/or the form of the IOL in the eye and also the dimension and/or relative position of a refractive index pattern already present, for generating a write pattern, wherein both the relative position and/or the form of the IOL in the eye and also the dimension and/or relative position of a refractive index pattern already present are taken into account, and for controlling the femtosecond pulse laser system to apply the write pattern to the IOL.

2. System for modifying the refractive index of an IOL, which has already been modified by means of a laser method, by a femtosecond laser, consisting of:
• a femtosecond pulse laser system comprising a femtosecond laser source, an optical unit suitable for focusing the laser beam into the eye and the IOL, and a scanner suitable for scanning the focal point of the laser beam laterally and axially with respect to the optical axis of the optical unit;
• a confocal detection measurement/imaging system comprising a light source/detector and a lateral scanner suitable for guiding the light of the light source into the eye via the same optical unit as the femtosecond pulse laser beam, the light reflected by the eye or the internal structures thereof being detected by the detector, wherein additional optical elements are present in the confocal detection system for the purposes of realizing a differential interference contrast phase contrast,
**characterized by**
a controller suitable for analysing the signals of the confocal system in order to capture both the relative position and/or the form of the IOL in the eye and also the dimension and/or relative position of a refractive index pattern already present, for generating a write pattern for the IOL, wherein both the relative position and/or the form of the IOL in the eye and also the dimension and/or relative position of a refractive index pattern already present are taken into account, and for controlling the laser system to apply the write pattern to the IOL.

3. System for modifying the refractive index of an IOL, which has already been modified by means of a laser method, by a femtosecond laser, consisting of:
• a femtosecond pulse laser system comprising a femtosecond laser source, an optical unit suitable for focusing the laser beam into the eye and the IOL, and a scanner suitable for scanning the focal point of the laser beam laterally and axially with respect to the optical axis of the optical unit;
**characterized by**
a 2D measurement/imaging system consisting of a camera suitable for taking at least two recordings of the eye, and hence of the IOL, at least two different illumination angles and illumination sources for the illumination thereof, wherein the 2-D measurement/imaging system contains additional optical elements for realizing a phase-contrast image and
• a controller suitable for analysing the signals of the camera in order to capture both the relative position and/or the form of the IOL in the eye and also the dimension and/or relative position of a refractive index pattern already present, for generating a write pattern for the IOL, wherein both the relative position and/or the form of the IOL in the eye and also the dimension and/or relative position of a refractive index pattern already present are taken into account, and for controlling the laser system to apply the write pattern to the IOL.

4. System for modifying the refractive index of an IOL, which has already been modified by means of a laser method, by a femtosecond laser, consisting of:
• a femtosecond pulse laser system comprising a femtosecond laser source, an optical unit suitable for focusing the laser beam into the eye , and a scanner suitable for scanning the focal point of the laser beam laterally and axially with respect to the optical axis of the optical unit;
• an optical coherence tomography (OCT) measurement/imaging system comprising an OCT light source/detector and a lateral scanner suitable for guiding the light of the OCT light source into the eye via the same optical unit as the femtosecond pulse laser beam and the OCT detector is suitable for detecting a light reflected by the eye or the internal structures thereof,
• a confocal detection measurement/imaging system comprising a light source/detector and a lateral scanner suitable for guiding the light of the light source into the eye via the same optical unit as the femtosecond pulse laser beam and the detector is suitable for detecting a light reflected by the eye or the internal structures thereof,
**characterized by**
a controller suitable for analysing the signals of the confocal system in order to capture the relative position and/or the form of the IOL in the eye and for analysing the signals of the OCT system in order to capture the relative position and extent of a refractive index pattern within the IOL in 3 dimensions, wherein the controller is further suitable for generating a write pattern, wherein both the relative position and/or the form of the IOL in the eye and also the dimension and/or relative position of a refractive index pattern already present are taken into account, and for controlling the laser system to apply the write pattern to the IOL.

5. System according to Claim 4, wherein the optical unit of the femtosecond pulse laser system comprising the femtosecond laser source is itself a scanning element.

6. System according to Claim 4, wherein the lateral scanner of the optical coherence tomography (OCT) measurement/imaging system is identical to the lateral scanner for deflecting the femtosecond laser.

7. System according to any one of Claims 1 to 4, wherein the control unit is embodied to control the laser system in such a way that a first or further write pattern of femtosecond laser pulses is applied into the IOL for the purposes of correcting a previously present multifocal structure of the IOL, at most 200 µm posterior or anterior to the previously present multifocal structure.

8. System according to any one of Claims 1 to 4, wherein the control unit is embodied to control the laser system on the basis of the IOL material in such a way that a further write pattern is applied to correct a previously present refractive index pattern.

9. System according to Claim 8, wherein the control unit is embodied to control the laser system in such a way that further write patterns are applied anterior to the previously present refractive index patterns, axially with respect to the eye axis.

10. System according to Claim 8, wherein the control unit is embodied to control the laser system in such a way that further write patterns are applied around already existing refractive index patterns or embedded in the latter.

11. System according to Claim 10, wherein the control unit is embodied to control the laser system in such a way that the present refractive index pattern and the complemented write pattern together yield a geometrically simple, contiguous refractive index pattern with a non-refractive optical power that does not correspond to a wrapped lens either.

12. System according to Claim 10, wherein the control unit is embodied to control the laser system in such a way that the write pattern and the present refractive index pattern overlap.

13. System according to Claim 8, wherein the control unit is embodied to control the laser system in such a way that the present refractive index pattern is deleted by the write pattern.

14. System according to Claim 8, wherein the control unit is embodied to control the laser system in such a way that the write pattern completely conceals the present refractive index pattern and additionally also covers parts of the previously unwritten IOL region.

15. System according to Claim 4, wherein the control unit is embodied to
• use OCT signals to calculate the relative position of the present refractive index pattern and the relative position of the IOL with respect to the coordinate system of an OCT system,
• use confocal detection signals to calculate the relative position of the IOL with respect to the coordinate system of a confocal detection system,
• calculate a coordinate transformation from the relative IOL position with respect to the coordinate system of the OCT system and the relative IOL position with respect to the coordinate system of the confocal detection system,
• convert the relative position of the present refractive index pattern with respect to the coordinate system of the OCT system into a relative position of the present refractive index pattern with respect to the coordinate system of the confocal detection system on the basis of the coordinate transformation and
• take into account the relative position data of the IOL and of the present refractive index pattern with respect to the coordinate system of the confocal detection system for the purposes of calculating the write pattern.

16. System according to any one of Claims 1 to 4, wherein the control unit is embodied to generate and apply one or more write patterns in order to convert
• a mono-focal IOL into a multi-focal IOL or
• a multi-focal IOL into a mono-focal IOL or
• a non-prismatic IOL into an IOL with an additional prismatic effect.

## Revendications

1. Système pour la modification d'un indice de réfraction d'une IOL, déjà modifiée au moyen d'un procédé laser, par un laser femtoseconde, composé :
• d'un système laser à impulsion femtoseconde avec une source laser femtoseconde, un dispositif optique qui est adapté pour focaliser le faisceau laser sur l'œil et l'IOL, et un scanner qui est adapté pour scanner le point de focalisation du faisceau laser latéralement et axialement à l'axe optique du dispositif optique ;
• d'un système de mesure/d'imagerie à tomographie par cohérence optique (OCT) comprenant une source de lumière/ un détecteur OCT et un scanner latéral qui est adapté pour guider dans l'œil la lumière de la source de lumière OCT en passant par le même dispositif optique que le faisceau laser à impulsion femtoseconde, le détecteur OCT étant adapté pour détecter à partir de la lumière réfléchie par l'œil ou ses structures internes un motif d'indice de réfraction et pour détecter la modification de phase par ce motif d'indice de réfraction ;
**caractérisé par** un appareil de commande qui est adapté pour analyser des signaux du système OCT, afin d'acquérir à la fois la position et/ou la forme de l'IOL dans l'œil et la dimension et/ou la position d'un motif d'indice de réfraction préexistant, pour générer un motif d'écriture dans lequel à la fois la position et/ou la forme de l'IOL dans l'œil et la dimension et/ou la position d'un motif d'indice de réfraction préexistant sont pris en compte ; et pour commander le système laser à impulsion femtoseconde afin d'appliquer le motif d'écriture à l'IOL.

2. Système pour la modification d'un indice de réfraction d'une IOL, déjà modifiée au moyen d'un procédé laser, par un laser femtoseconde, composé :
• d'un système laser à impulsion femtoseconde avec une source laser femtoseconde, un dispositif optique qui est adapté pour focaliser le faisceau laser sur l'œil et l'IOL, et un scanner qui est adapté pour scanner le point de focalisation du faisceau laser latéralement et axialement à l'axe optique du dispositif optique ;
• d'un système de mesure/d'imagerie à détection confocale, comprenant une source/un détecteur laser et un scanner latéral qui est adapté pour guider dans l'œil la lumière de la source de lumière en passant par le même dispositif optique que le faisceau laser à impulsion femtoseconde, la lumière réfléchie par l'œil ou ses structures internes étant détectée par le détecteur, dans lequel il existe des éléments optiques supplémentaires dans le système de détection confocale pour réaliser un contraste d'interférence différentielle ou un contraste de phase,
**caractérisé par** un appareil de commande qui est adapté pour analyser des signaux du système confocal pour acquérir à la fois la position et/ou la forme de l'IOL dans l'œil et la dimension et/ou la position d'un motif d'indice de réfraction préexistant, pour générer un motif d'écriture pour l'IOL, dans lequel à la fois la position et/ou la forme de l'IOL dans l'œil et la dimension et/ou la position d'un motif d'indice de réfraction préexistant sont prises en compte ; et pour commander le système laser pour appliquer le motif d'écriture à l'IOL.

3. Système pour la modification d'un indice de réfraction d'une IOL, déjà modifiée au moyen d'un procédé laser, par un laser femtoseconde, composé :
• d'un système laser à impulsion femtoseconde avec une source laser femtoseconde, un dispositif optique qui est adapté pour focaliser le faisceau laser sur l'œil et l'IOL, et un scanner qui est adapté pour scanner le point de focalisation du faisceau laser latéralement et axialement à l'axe optique du dispositif optique ;
**caractérisé par** un système de mesure/d'imagerie 2D composé d'une caméra qui est adaptée pour prendre au moins deux clichés de l'œil et donc de l'IOL selon au moins deux angles d'éclairage différents, et des sources d'éclairage pour l'éclairer, le système de mesure/d'imagerie 2D contenant de plus des éléments optiques pour réaliser une image à contraste de phase, et
• un appareil de commande qui est adapté pour analyser des signaux de la caméra afin d'acquérir à la fois la position et/ou la forme de l'IOL dans l'œil et la dimension et/ou la position d'un motif d'indice de réfraction préexistant, pour générer un motif d'écriture pour l'IOL, dans lequel à la fois la position et/ou la forme de l'IOL dans l'œil et la dimension et/ou la position d'un motif d'indice de réfraction préexistant sont prises en compte ; et pour commander le système laser pour appliquer le motif d'écriture à l'IOL.

4. Système pour la modification d'un indice de réfraction d'une IOL, déjà modifiée au moyen d'un procédé laser, par un laser femtoseconde, composé :
• d'un système laser à impulsion femtoseconde avec une source laser femtoseconde, un dispositif optique qui est adapté pour focaliser le faisceau laser sur l'œil et l'IOL, et un scanner qui est adapté pour scanner le point de focalisation du faisceau laser latéralement et axialement à l'axe optique du dispositif optique ;
• d'un système de mesure/d'imagerie à tomographie par cohérence optique (OCT) comprenant une source de lumière/ un détecteur OCT et un scanner latéral qui est adapté pour guider la lumière de la source de lumière OCT dans l'œil en passant par le même dispositif optique que le faisceau laser à impulsion femtoseconde, et le détecteur OCT est adapté pour détecter la lumière réfléchie par l'œil ou ses structures internes ;
• d'un système de mesure/d'imagerie à détection confocale comprenant une source de lumière/ un détecteur et un scanner latéral qui est adapté pour guider la lumière de la source de lumière dans l'œil en passant par le même dispositif optique que le faisceau laser à impulsion femtoseconde ; et le détecteur est adapté pour détecter la lumière réfléchie par l'œil ou ses structures internes ;
**caractérisé par** un appareil de commande qui est adapté pour analyser des signaux du système confocal, pour acquérir la position et/ou la forme de l'IOL dans l'œil, et pour analyser les signaux du système OCT pour acquérir la position, l'étendue d'un motif d'indice de réfraction à l'intérieur de l'IOL en 3 dimensions, l'appareil de commande étant en outre adapté pour générer un motif d'écriture, dans lequel à la fois la position et/ou la forme de l'IOL dans l'œil et l'étendue et la position d'un motif d'indice de réfraction préexistant sont prises en compte ; et le système laser est adapté pour commander l'application du motif d'écriture à l'IOL.

5. Système selon la revendication 4, dans lequel le dispositif optique du système laser à impulsion femtoseconde avec une source laser femtoseconde est lui-même un élément de balayage.

6. Système selon la revendication 4, dans lequel le scanner latéral du système de mesure/d'imagerie de tomographie par cohérence optique (OCT) est identique au scanner latéral destiné à dévier le laser femtoseconde.

7. Système selon l'une quelconque des revendications 1 à 4, dans lequel l'unité de commande est réalisée pour commander le système laser de telle sorte qu'un premier motif d'écriture ou un motif d'écriture supplémentaire d'impulsions de laser femtoseconde est appliqué à l'IOL pour corriger une structure multifocale préexistante de l'IOL, au plus postérieure ou antérieure de 200 µm à la structure multifocale préexistante.

8. Système selon l'une quelconque des revendications 1 à 4, dans lequel l'unité de commande est réalisée pour commander le système laser en fonction du matériau d'IOL de telle sorte qu'un motif d'écriture supplémentaire est appliqué pour corriger un motif d'indice de réfraction préexistant.

9. Système selon la revendication 8, dans lequel l'unité de commande est réalisée pour commander le système laser de telle sorte que des motifs d'écriture supplémentaires sont appliqués axialement par rapport à l'axe de l'œil de manière antérieure aux motifs d'indice de réfraction préexistants.

10. Système selon la revendication 8, dans lequel l'unité de commande est réalisée pour commander le système laser de telle sorte que des motifs d'écriture supplémentaires sont appliqués autour de motifs d'indice de réfraction préexistants ou sont incorporés dans ceux-ci.

11. Système selon la revendication 10, dans lequel l'unité de commande est réalisée pour commander le système laser de telle sorte que le motif d'indice de réfraction préexistant et le motif d'écriture complété produisent ensemble un motif d'indice de réfraction cohérent, géométriquement simple, ayant un effet optique non diffractif et ne correspondant pas à une lentille enveloppée.

12. Système selon la revendication 10, dans lequel l'unité de commande est réalisée pour commander le système laser de telle sorte que le motif d'écriture et le motif d'indice de réfraction existant se chevauchent.

13. Système selon la revendication 8, dans lequel l'unité de commande est réalisée pour commander le système laser de telle sorte que le motif d'indice de réfraction existant est supprimé par le motif d'écriture.

14. Système selon la revendication 8, dans lequel l'unité de commande est réalisée pour commander le système laser de telle sorte que le motif d'écriture recouvre complètement le motif d'indice de réfraction existant et recouvre de plus aussi des parties de la zone d'IOL précédemment sans écriture.

15. Système selon la revendication 4, dans lequel l'unité de commande est réalisée pour
• utiliser des signaux OCT pour calculer la position du motif d'indice de réfraction existant et la position de l'IOL par rapport au système de coordonnées d'un système OCT,
• utiliser des signaux de détection confocale pour calculer la position de l'IOL par rapport au système de coordonnées d'un système de détection confocale,
• calculer une transformation de coordonnées à partir de la position d'IOL par rapport au système de coordonnées du système OCT et de la position d'IOL par rapport au système de coordonnées du système de détection confocale,
• convertir la position du motif d'indice de réfraction existant par rapport au système de coordonnées du système OCT à l'aide de la transformation de coordonnées en une position du motif d'indice de réfraction existant par rapport au système de coordonnées du système de détection confocale, et
• prendre en compte pour le calcul du motif d'écriture des données de position de l'IOL et du motif d'indice de réfraction existant par rapport au système de coordonnées du système de détection confocale.

16. Système selon l'une quelconque des revendications 1 à 4, dans lequel l'unité de commande est réalisée pour produire et appliquer un ou plusieurs motifs d'écriture, pour transformer
• une IOL monofocale en une IOL multifocale, ou
• une IOL multifocale en une IOL monofocale, ou
• une IOL non prismatique en une IOL ayant un effet prismatique supplémentaire.
